# EUROPEAN PATENT SPECIFICATION

(11) **EP 3 222 246 B1**
(45) Date of publication and mention of the grant of the patent: **15.12.2021**
(21) Application number: 17162663.3
(22) Date of filing: 23.03.2017
(51) Int. Cl.: A61C 17/02, A61M 5/31, A61C 5/62

(54) **DENTAL SYRINGE HOLDER AND SYRINGE EQUIPPED WITH DENTAL SYRINGE HOLDER**
HALTER FÜR ZAHNÄRZTLICHE SPRITZE UND MIT DEM HALTER FÜR ZAHNÄRZTLICHE SPRITZEN AUSGESTATTETE SPRITZE
SUPPORT DE SERINGUE DENTAIRE ET SERINGUE ÉQUIPÉE D'UN SUPPORT DE SERINGUE DENTAIRE

(30) Priority: 24.03.2016 JP 2016060944
(43) Date of publication of application: 27.09.2017
(73) Proprietor: SHOFU INC., Kyoto 605-0983 (JP)
(72) Inventor: UCHIDA, Jun, KYOTO, 605-0983 (JP); FUJIMURA, Hidefumi, KYOTO, 605-0983 (JP)
(74) Representative: Eisenführ Speiser

(56) References cited:
- CN-U- 204 618 251
- DE-A1- 3 910 982
- US-A- 4 909 788
- US-A- 5 453 093

## Description

### BACKGROUND OF THE INVENTION

### Field of the Invention

The present invention relates to a system comprising a dental syringe and a dental syringe holder, and more particularly a dental syringe holder that is charged with paste for prosthesis (or restoration).

### Description of the Related Art

As the treatment method for recovering a reduced oral function due to tooth loss, a prosthesis restoration treatment is cited. In the prosthesis restoration treatment, a prosthesis in the shape of a tooth crown is produced from a material such as a dental metal, porcelain, a composite resin or ceramics, and the produced prosthesis is bonded and fused to a site to be restored. As a more specific example, an outline of a process in the case of producing a facing crown will be shown. Note that a facing crown refers to a tooth crown restoration that recovers not only the function in an oral cavity but also dental aesthetics by producing a part that is exposed to external view by a tooth crown material such as a resin material, of a coverage crown.

First, appropriate pretreatment such as sandblast is applied to a metal casting called a metal frame, and the treated surface is coated with a metal adhesive primer by a brush or the like.

Pretreatment of forming unevenness may be applied to the surface of the metal frame by attaching retention beads to the surface of the metal frame to enhance a mechanical bonding force to the resin. In this case, in order to fill gaps among the plurality of retention beads and give bonding strength, a material called a preopaque material is coated and cured. Subsequently, to hide a metallic color, opaque paste formed from a curable composition having a high contrast ratio is coated and cured. Coating and curing of the opaque paste are repeated in accordance with necessity. After sufficient metal shielding performance is obtained, a target tooth crown restoration is coated with paste of a coloring material for which a color tone is adjusted, by using a brush or the like in accordance with necessity, after which, a tooth crown forming rigid resin paste is built up on the target tooth crown restoration little by little, while stacking is repeated in accordance with necessity, and a tooth shape is produced ultimately.

In the dental treatment of recent years, importance of aesthetics has been increased, and aesthetics at a high level has been required in the aforementioned facing crown in particular, so that in place of coating an opaque material and paste of a coloring material by brushes or the like, these pastes have been directly built up from dental syringes charged with these pastes. In order to enhance working efficiency of the work of building up paste as above, it is necessary to hold a dental syringe stably. As a holder for holding a dental syringe stably, there is a handle for crea.lign Dentin that is sold by bredent medical GmbH & Co. KG, for example (http://www.bredent.com/en/bredent/product-group/155005/page/1/).

In the work of building up of paste or the like by using a dental syringe, rotation of the dental syringe, and precise work of adjustment of a space from an object to be worked such as a prosthesis are important. In the conventional dental syringe holder in Non Patent Literature 1, the dental syringe is rotatable with a thumb and a forefinger and/or a middle finger, but in order to hold the dental syringe holder stably, it is necessary to grip a gripping portion that is formed on an outer periphery of the syringe holding portion that holds the syringe by using a middle finger, a third finger (a ring finger), and a little finger, and therefore the middle finger, the third finger and the little finger cannot be used in adjustment of the space from the object to be worked such as a prosthesis. Consequently, in the conventional dental syringe holder in Non Patent Literature 1, adjustment of the space from the object to be worked such as a prosthesis cannot be sufficiently performed, and a case arises, in which workability of precise work in the dental syringe cannot be enhanced.

The present invention is made to solve the problem as described above, and has an object to provide a dental syringe holder that can enhance workability of precise work in a dental syringe while enhancing stability in holding the dental syringe.

CN 204618251 U describes a needle holder for venous blood collection, comprising a fixing base, which holds a cylinder. The fixing base configured to be fixed on the surface of human skin of the arm for drawing blood.

US 5,453,093 discloses a disposable dental syringe.

### SUMMARY OF THE INVENTION

The inventors have found out that in the work of building up of paste or the like by using a dental syringe, it is possible to achieve compatibility between keeping stability in holding the dental syringe, and enhancement of workability of precise work in the dental syringe as much as possible by making the dental syringe turnable with a thumb and a forefinger while holding the dental syringe with three fingers that are the thumb, the forefinger and a middle finger in such a manner as to hold a pen, making a finger that is caused to abut on an object to be worked selectable from three fingers that are the middle finger, a third finger and a little finger, and increasing the range in which the space between the dental syringe and the object to be worked can be adjusted.

As a result that the present inventors made earnest study on the dental syringe holder in the case of holding the dental syringe in such a manner as to hold a pen based on these findings, the present inventors have found out that workability of precise work in the dental syringe can be enhanced while stability in holding the dental syringe held by the dental syringe holder is enhanced, by forming a clamping wing portion that is clamped with the forefinger and the middle finger on the outer periphery of the syringe holding portion, and clamping the clamping wing portion with the forefinger and the middle finger.

The present invention is based on these findings, and the configuration of the present invention is as defined in the appended claims.

In the system of the invention, a dental syringe holder includes a syringe holding portion that attachably and detachably holds an outer barrel of the dental syringe, and a clamping wing portion that is formed on an outer periphery of the syringe holding portion, and can be clamped with a forefinger and a middle finger in the case of holding the dental syringe with three fingers that are the thumb, the forefinger and a middle finger in such a manner as to hold a pen.

Thereby, workability of precise work in a dental syringe can be enhanced while stability in holding the dental syringe is enhanced.

Further, the dental syringe holder includes a placing wing portion which is formed on the outer periphery of the syringe holding portion, and on which a thumb can be placed in the case of holding the dental syringe with three fingers that are the thumb, the forefinger and a middle finger in such a manner as to hold a pen.

According to the configuration, workability of precise work in the dental syringe can be further enhanced while stability in holding the dental syringe is further enhanced.

Further, in the dental syringe holder, in a projected image on a plane orthogonal to an axial direction of the syringe, an angle that is formed by a first line segment connecting a center of a minimum virtual circle surrounding the outer periphery of the syringe holding portion and a formation position of the clamping wing portion, and a second line segment connecting the center of the virtual circle and a formation position of the placing wing portion is in a range from 90° to 170° inclusive.

According to the configuration, workability of precise work in the dental syringe can be further enhanced while stability in holding the dental syringe is further enhanced.

Further, in the dental syringe holder, a maximum thickness dimension of the clamping wing portion is preferably 0.2 mm to 5 mm inclusive.

According to the configuration, workability of precise work in the dental syringe can be further enhanced while stability in holding the dental syringe is further enhanced.

Here, the maximum thickness dimension of the clamping wing portion is a maximum dimension of a shortest distance between a surface which the forefinger contacts, and a surface which the middle finger contacts.

Further, in the dental syringe holder, a maximum thickness dimension of the clamping wing portion and a maximum thickness dimension of the placing wing portion are preferably 0.2 mm to 5 mm inclusive.

According to the configuration, workability of precise work in the dental syringe can be further enhanced while stability in holding the dental syringe is further enhanced.

Here, the maximum thickness dimension of the placing wing portion is a maximum dimension of a shortest distance between a surface on which the thumb is placed, and a surface which faces the surface on which the thumb is placed.

Further, in the dental syringe holder, the dental syringe holder preferably holds the syringe slidably.

According to the configuration, workability of precise work in the dental syringe can be further enhanced while stability in holding the dental syringe is further enhanced.

Further, the dental syringe and the dental syringe holder are included in the system of the present invention.

According to the present invention, the dental syringe holder can be provided, which can enhance workability of precise work in the dental syringe while enhancing stability in holding the dental syringe.

### BRIEF DESCRIPTION OF THE DRAWINGS

FIG. 1 is a schematic side view of a dental syringe holder for use in a system according to one embodiment of the present invention;
FIG. 2 is a schematic side view of a dental syringe holder in which a syringe holding portion and a cap portion are separable;
FIG. 3A is a plan view illustrating an inner circumferential surface side of the syringe holding portion;
FIG. 3B is a left side (side at one end side) view of the syringe holding portion;
FIG. 4A is a plan view illustrating an inner circumferential surface side of the cap portion;
FIG. 4B is a left side view of the cap portion; and
FIG. 5 is a view illustrating a use state of the dental syringe holder for use in a system according to one embodiment.

### DETAILED DESCRIPTION OF THE PREFERRED EMBODIMENTS

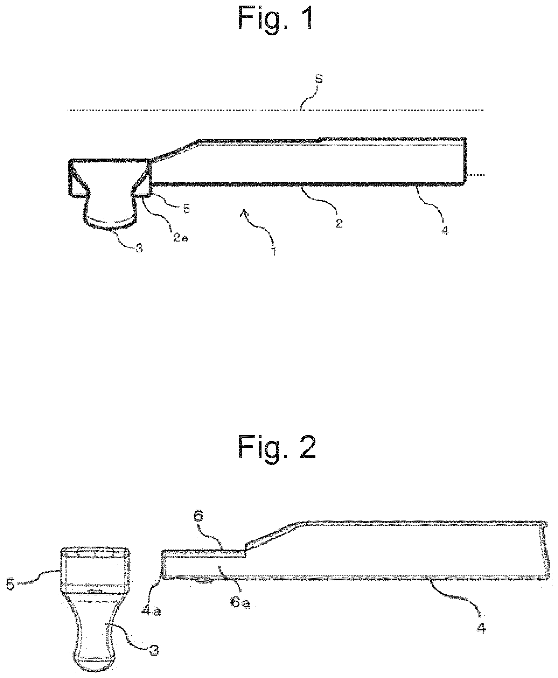

Hereunder, an embodiment of the present invention will be illustrated and described in detail with reference to the drawings. FIG. 1 is a schematic side view of an integrated type dental syringe holder 1 for use in a system according to one embodiment of the present invention. FIG. 2 is a schematic side view of a separate type dental syringe holder 1 in which a syringe holding portion 4 and a cap portion 5 are separable. Hereunder, the separable dental syringe holder 1 in FIG. 2 will be illustrated and described in detail. The dental syringe holder 1 is formed of the syringe holding portion 4 that holds an outer barrel of a syringe S attachably and detachably, and the cap portion 5 that can be fitted to an outer circumferential surface 6a of the syringe holding portion 4, and a pair of finger laying portions 3 are formed on the cap portion 5. In the pair of finger laying portions 3 that are formed on the cap portion 5, a clamping wing portion 3a and a placing wing portion 3b are formed. The clamping wing portion 3a is a clamping wing portion that is clamped with a forefinger and a middle finger, and the placing wing portion 3b is a placing wing portion on which a thumb is placed. Note that the clamping wing portion may be clamped by a combination of fingers except for the combination of a forefinger and a middle finger. Likewise, on the placing wing portion, a finger except for a thumb may be placed.

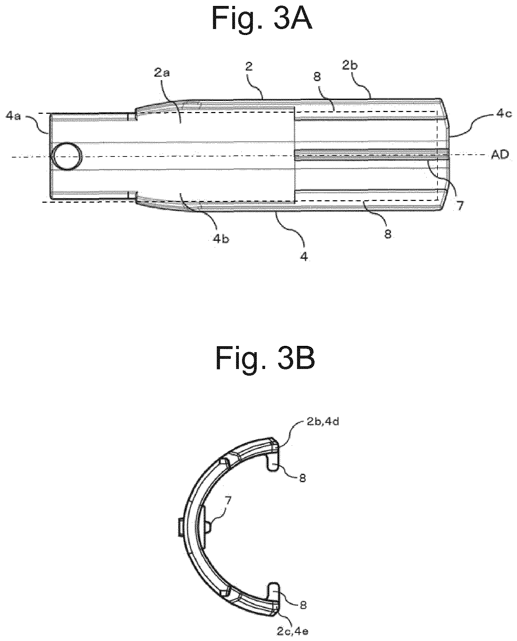

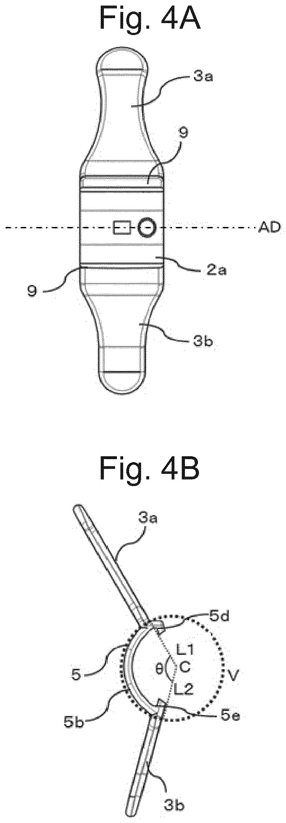

FIGS. 3A and 3B are a plan view and a left side view of the syringe holding portion 4, and FIGS. 4A and 4B are a plan view and a left side view of the cap portion 5. In the present embodiment, the syringe holding portion 4 has a narrow width portion 6 to and from which the cap portion 5 can be attached and detached, at one end 4a side. In the present embodiment, the cap portion 5 is slid in a longitudinal direction of the syringe holding portion 4 from the one end 4a of the syringe holding portion 4, in such a manner that the outer circumferential surface 6a of the narrow width portion 6 and an inner circumferential surface 5a of the cap portion 5 come into contact with each other, whereby the cap portion 5 can be fitted to the syringe holding portion 4.

In the clamping wing portion 3a, a dimension in a longitudinal direction in a projected image on a plane orthogonal to an axial direction of a syringe S (a dimension to a tip end from a connection portion to an outer circumferential surface 5b of the cap portion 5) is preferably 1 cm to 7 cm inclusive. In a case of being less than 1 cm, it becomes difficult to clamp the clamping wing portion 3a with a forefinger and a middle finger, and a case arises, in which stability in holding the dental syringe cannot be sufficiently obtained. Further, in a case of being larger than 7 cm, the clamping wing portion 3a interferes with work of building up or the like, and a case can arise, in which workability of precise work in the dental syringe cannot be enhanced.

Further, a width dimension (a dimension from one end to the other end in the axial direction of the syringe S, in the outer circumferential surface 5b of the cap portion 5) of the clamping wing portion 3a is preferably 0.1 cm to 2 cm inclusive. In a case of being less than 0.1 cm, a contact area of a forefinger and a middle finger, and the clamping wing portion 3a cannot be ensured, and a case arises, in which stability in holding the dental syringe cannot be sufficiently obtained. Further, in a case of being larger than 2 cm, the clamping wing portion 3a interferes with work of building up or the like, and a case can arise, in which workability of precise work in the dental syringe cannot be enhanced.

Furthermore, a maximum thickness dimension (a maximum dimension of a shortest distance between a surface which a forefinger contacts and a surface which a middle finger contacts) of the clamping wing portion 3a is preferably 0.2 mm to 5 mm inclusive. In a case of being less than 0.2 mm, strength of the clamping wing portion 3a becomes insufficient, and breakage, deformation and the like of the clamping wing portion 3a can occur. Further, in a case of being larger than 5 mm, a sense of discomfort occurs in the feeling of clamping with a forefinger and a middle finger, and work of building up or the like becomes difficult.

In the placing wing portion 3b, a dimension in a longitudinal direction in the projected image on the plane orthogonal to the axial direction of the syringe S (a dimension to a tip end from a connection portion to the outer circumferential surface 5b of the cap portion 5) is preferably 0.5 cm to 5 cm inclusive. In a case of being less than 0.5 cm, it becomes difficult to place a thumb sufficiently, and a case arises, in which stability in holding the dental syringe cannot be sufficiently obtained. Further, in a case of being larger than 5 cm, the placing wing portion 3b interferes with work of building up or the like, and workability of precise work in the dental syringe cannot be enhanced.

Further, a width dimension (a dimension from one end to the other end in the axial direction of the syringe S in the outer circumferential surface 5b of the cap portion 5) of the placing wing portion 3b is preferably 0.2 cm to 3 cm inclusive. In a case of being less than 0.2 cm, a contact area of a thumb, and the placing wing portion 3b cannot be ensured, and stability in holding the dental syringe cannot be sufficiently obtained. Further, in a case of being larger than 3 cm, the placing wing portion 3b interferes with work of building up or the like, and workability of precise work in the dental syringe cannot be enhanced.

Furthermore, a maximum thickness dimension (a maximum dimension of a shortest distance between a surface on which a thumb is placed and a facing surface) of the placing wing portion 3b is preferably 0.2 mm to 5 mm inclusive. In a case of being less than 0.2 mm, strength of the placing wing portion 3b becomes insufficient, and breakage, deformation and the like can occur to the placing wing portion 3b. Further, in a case of being larger than 5 mm, a sense of discomfort occurs in the feeling of placing a thumb, and work of building up or the like becomes difficult.

As illustrated in FIGS. 3A and 3B and FIGS. 4A and 4B, the dental syringe holder 1 for use in a system of the present embodiment has a cutout cylinder shape. Specifically, in the present embodiment, the syringe holding portion 4 and the cap portion 5 that form the dental syringe holder 1 both have cutout cylinder shapes.

As illustrated in FIG. 3A, in the present embodiment, a protruding portion 7 is formed on an inner circumferential surface 4b of the syringe holding portion 4 that forms the dental syringe holder 1. In detail, the protruding portion 7 of the present embodiment has a trapezoidal shape in section as illustrated in FIGS. 3A and 3B, and extends to a vicinity of a center of the syringe holding portion 4 along the longitudinal direction of the syringe holding portion 4 from the other end 4c of the syringe holding portion 4.

As illustrated in FIGS. 3A and 3B, in the present embodiment, protruded portions 8 are respectively formed in regions to be both ends 4d and 4e in a projected image on a plane orthogonal to an axial direction AD of the syringe S, in the inner circumferential surface 4b of the syringe holding portion 4. The protruded portion 8 in the present embodiment extends to the vicinity of the center of the syringe holding portion 4 along the longitudinal direction of the syringe holding portion 4 from the other end 4c of the syringe holding portion 4, as illustrated in FIGS. 3A and 3B. In the present embodiment in particular, a dimension of the protruding portion 7 along the longitudinal direction of the syringe holding portion 4 and a dimension of the protruded portion 8 along the longitudinal direction of the syringe holding portion 4 are configured to be the same.

The protruded portion 8 is preferably formed to contact an outer barrel of the syringe S. Further, when a slit for attaching the dental syringe holder 1 is formed in the syringe S, the protruded portion 8 is more preferably formed to be fitted in the slit.

As illustrated in FIGS. 4A and 4B, in the present embodiment, in the projected image on the plane orthogonal to the axial direction AD of the syringe S, an angle θ that is formed by a first line segment L1 connecting a center C of a minimum virtual circle V that surrounds an outer periphery of the syringe holding portion 4 and a first formation position P1 of the clamping wing portion 3a, and a second line segment L2 connecting the center C of the virtual circle V and a second formation position P2 of the placing wing portion 3b is in a range from 90° to 170° inclusive. Specifically, in the present embodiment, the angle formed by the line segment L1 and the line segment L2 is 165°.

In detail, in the present embodiment, the clamping wing portion 3a is formed from an end 5c to an end 5d of an outer circumferential surface 5b of the cap portion 5 that forms the dental syringe holder 1. Furthermore, in the present embodiment, the placing wing portion 3b is formed from an end 5e to an end 5f of the outer circumferential surface 5b of the cap portion 5 that forms the dental syringe holder 1. Here, a shape of the outer circumferential surface 6a of the narrow width portion 6 in the projected image on the plane orthogonal to the axial direction AD of the syringe S is the same as a shape of the inner circumferential surface 5a of the cap portion 5 in FIG. 4B.

Note that in the present embodiment, the outer circumferential surface 5b in a circular arc shape of the cap portion 5 is cut out at the end 5d and the end 5e.

As illustrated in FIGS. 4A and 4B, in the present embodiment, the clamping wing portion 3a and the placing wing portion 3b differ from each other in dimension in the longitudinal direction. Specifically, a dimension in the longitudinal direction of the clamping wing portion 3a is configured to be longer than a dimension in the longitudinal direction of the placing wing portion 3b.

In the present embodiment in particular, as illustrated in FIGS. 4A and 4B, protruded portions 9 are respectively formed from the end 5c to the end 5d and from the end 5e to the end 5f, on the inner circumferential surface 5a of the cap portion 5. Consequently, in spaces sandwiched by the inner circumferential surface 5a of the cap portion 5 and the protruded portions 9, both ends in the projected image on the plane orthogonal to the axial direction AD of the syringe S, of the narrow width portion 6 of the syringe holding portion 4 can be held.

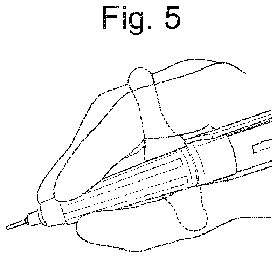

Next, a method for using the dental syringe holder 1 will be described. First, the cap portion 5 having a suitable pair of finger laying portions 3 is selected in accordance with an object to be worked, a worker and a work content, and the cap portion is slid from the one end 4a of the syringe holding portion 4 and fitted to the syringe holding portion 4, in such a manner that the protruded portions 9 formed at the cap portion 5 hold both the ends of the narrow width portion 6 of the syringe holding portion 4. Next, the syringe S is inserted from one end of the dental syringe holder 1, and the dental syringe holder 1 is caused to hold the syringe S in such a matter that the protruded portions 8 come into contact with the outer barrel of the syringe S or are fitted in the slits of the syringe S. As illustrated in FIG. 5, the clamping wing portion 3a is held between a forefinger and a middle finger, a thumb is placed on the placing wing portion 3b to hold the dental syringe holder 1, the syringe S is moved with respect to the dental syringe holder 1, and building up paste or the like is performed by turning the syringe S with the thumb and the forefinger, while causing one or more of the three fingers that are the middle finger, the third finger and the little finger to abut on the object to be worked in a desired mode.

Hereunder, an operational effect of the dental syringe holder 1 for use in a system of the present invention will be described with a case of holding the dental syringe with three fingers that are a thumb, a forefinger, and a middle finger in such a manner as to hold a pen cited as an example.

The dental syringe holder 1 for use in a system of the present invention includes the clamping wing portion 3a that can be clamped with the forefinger and the middle finger. First of all, in the dental syringe holder 1 of a configuration like this, the dental syringe S can be made rotatable with the thumb and the forefinger while the dental syringe S is held with the three fingers that are the thumb, the forefinger and the middle finger in such a manner as to hold a pen. Further, by clamping the clamping wing portion 3a with the forefinger and the middle finger, whereby stability in holding the dental syringe S held by the dental syringe holder 1 can be enhanced. Further, the dental syringe S can be stably held without using the three fingers that are a tip end side of the middle finger, the third finger and the little finger, and therefore, a finger that is caused to abut on the object to be worked can be selected from the middle finger, the third finger and the little finger. Consequently, a range in which a space between the dental syringe S and the object to be worked can be adjusted can be increased, and workability of precise work in the dental syringe S can be enhanced.

As above, according to the dental syringe holder 1 for use in a system of the present embodiment, workability of precise work in the dental syringe S can be enhanced while stability in holding the dental syringe S is kept.

In the present invention, as illustrated in FIGS. 2 to 4B, the placing wing portion 3b on which a thumb can be placed is included.

In doing so, shaking of the dental syringe holder 1 can be suppressed by placing a thumb on the placing wing portion 3b, and therefore, workability of precise work in the dental syringe can be further enhanced while stability in holding the dental syringe is further enhanced.

In the present invention, as illustrated in FIGS. 2 to 4B, in the projected image on the plane orthogonal to the axial direction AD of the syringe S, the angle θ which is formed by the first line segment L1 connecting the center C of the minimum virtual circle V that surrounds the outer periphery of the syringe holding portion 4 and the first formation portion P1 of the clamping wing portion 3a, and the second line segment L2 connecting the center C of the virtual circle V and the second formation position P2 of the placing wing portion 3b is preferably in a range from 90° to 170° inclusive. In doing so, the clamping wing portion 3a and the placing wing portion 3b are formed asymmetrically in the projected image on the plane orthogonal to the axial direction AD of the syringe S. Consequently, positions of the clamping wing portion 3a and the placing wing portion 3b can be caused to correspond to the relation of the position of the thumb and the position between the forefinger and the middle finger to the dental syringe in the case of holding the dental syringe S, and a positional relation of the thumb and the forefinger at the time of rotating the dental syringe with the thumb and the forefinger. Further, it becomes possible to use a middle finger in adjustment of the space from the object to be worked. Consequently, workability of precise work in the dental syringe can be further enhanced while stability in holding the dental syringe is enhanced.

In the present invention, as illustrated in FIGS. 2 to 4B, the maximum thickness dimension of the clamping wing portion 3a is preferably 0.2 mm to 5 mm inclusive. In doing so, occurrence of the case can be prevented, in which a sense of discomfort occurs in the feeling of clamping with a forefinger and a middle finger, and work of building up or the like becomes difficult, while breakage, deformation and the like of the clamping wing portion 3a are prevented. Consequently, workability of precise work in the dental syringe can be further enhanced while stability in holding the dental syringe is further enhanced.

In the present invention, as illustrated in FIGS. 2 to 4B, the maximum thickness dimension of the placing wing portion 3b is preferably 0.2 mm to 5 mm inclusive. In doing so, occurrence of the case can be prevented, in which a sense of discomfort occurs in the feeling of placing a thumb, and work of building up or the like becomes difficult, while breakage, deformation and the like of the placing wing portion 3b are prevented. Consequently, workability of precise work in the dental syringe can be further enhanced, while stability in holding the dental syringe is further enhanced.

In the present invention, as illustrated in FIGS. 2 to 4B, the syringe is preferably held slidably. In doing so, the space between the syringe S and the object to be worked can be adjusted, and therefore, at the time of performing building up of paste or the like, one or more of the three fingers that are the middle finger, the third finger and the little finger can be caused to abut on the object to be worked in a desired mode. Consequently, workability of precise work in the dental syringe can be further enhanced, while stability of holding the dental syringe is further enhanced.

In the present invention, as illustrated in FIGS. 2 to 4B, at least the one protruding portion 7 for ensuring a gap between the outer circumferential surface of the syringe S and the inner circumferential surface 4b of the syringe holding portion 4 is preferably formed on the inner circumferential surface 4b of the syringe holding portion 4 of the dental syringe holder 1. In doing so, a contact area of the outer circumferential surface of the syringe S and the inner circumferential surface 4b of the syringe holding portion 4 can be decreased, and therefore, rotation and movement of the syringe S to the syringe holder 1 can be easily performed.

Further, in the present invention, the dimension in the longitudinal direction of the clamping wing portion 3a is preferably longer than the dimension in the longitudinal direction of the placing wing portion 3b. By adopting the configuration like this, even when the middle finger moves in a direction to be away from the syringe to be used in adjustment of the space from the object to be worked, for example, the clamping wing portion 3a having a large dimension in the longitudinal direction can be clamped with the forefinger and the middle finger, and therefore, stability in holding the dental syringe is not impaired. Further, the placing wing portion 3b is more prevented from interfering with the work of building up or the like, and workability of precise work in the dental syringe can be more enhanced. Note that the dimension in the longitudinal direction of the clamping wing portion 3a and the dimension in the longitudinal direction of the placing wing portion 3b may be the same.

Further, in the present invention, the syringe holder 1 preferably has a cutout cylinder shape as illustrated in FIGS. 2 to 4B. By adopting the configuration like this, attachment and detachment of the dental syringe S can be easily performed.

Further, in the present invention, as illustrated in FIGS. 2 to 4B, on the inner circumferential surface 4b of the syringe holding portion 4, the protruded portions 8 that hold the syringe S are preferably formed in the regions to be both the ends 4d and 4e in the projected image on the plane orthogonal to the axial direction of the syringe S. According to the configuration, falling-off of the dental syringe can be prevented.

Further, in the present invention, as illustrated in FIGS. 2 to 4B, the placing wing portion 3b is preferably formed from the end 5c to the end 5d of the cap portion 5, and the clamping wing portion 3a is preferably formed from the end 5e to the end 5f of the cap portion. According to the configuration, it becomes easier to use the middle finger in adjustment of the space from the object to be worked, while optimizing the positions of the placing wing portion 3b and the clamping wing portion 3a, to the relation of the position of the thumb and the position between the forefinger and the middle finger to the dental syringe in the case of holding the dental syringe, and the positional relation of the thumb and the forefinger at the time of turning the dental syringe with the thumb and the forefinger. Consequently, according to the configuration, workability of precise work in the dental syringe can be further enhanced.

Furthermore, in the present invention, as illustrated in FIGS. 2 to 4B, the syringe holder 1 has the syringe holding portion 4 and the cap portion 5 that is attachable to and detachable from the one end 4a of the narrow width portion 6, and the pair of finger laying portions 3 are preferably formed at the cap portion 5. According to the configuration, even when change of the object to be worked, change of a worker, and change of a hand which is used by the worker are present, the pair of finger laying portions 3 can be optimized by changing only the cap portion 5, and therefore, workability of precise work in the dental syringe can be further enhanced while stability in holding the dental syringe is further enhanced.

In the above described one embodiment, the case in which the syringe holder 1 is separated to be the syringe holding portion 4 and the attachable and detachable cap portion 5 is described, but the whole of the syringe holder 1 may be integrally formed as in FIG. 1.

As the dental syringe holder 1 for use in a system according to another embodiment, the syringe holding portion 4 can be formed into a cylindrical shape. In the present embodiment, falling-off of the dental syringe can be prevented with the protruding portions 7 and the protruded portions 8 while stability in holding the dental syringe and workability of precise work in the dental syringe are enhanced.

In the dental syringe holder for use in a system according to still another embodiment of the present invention, the clamping wing portion 3a and the placing wing portion 3b can be also formed as ring-shaped finger laying portions. In the present configuration, stability in holding the dental syringe is enhanced, and workability of precise work in the dental syringe is enhanced by adopting the ring-shaped clamping wing portion 3a and placing wing portion 3b.

The present invention can be grasped as a dental syringe equipped with the dental syringe holder of the present invention.

The present invention can enhance stability in holding the dental syringe at the time of producing a dental prosthesis.

## Claims

1. A system comprising:
a dental syringe (S); and
a dental syringe holder (1), comprising:
a syringe holding portion (4) that attachably and detachably holds an outer barrel of the dental syringe (S); and
a clamping wing portion (3a) that is formed on an outer periphery of the syringe holding portion (5), and is configured to be clamped by a forefinger and a middle finger in the case of holding the dental syringe (S) with three fingers that are the thumb, the forefinger and a middle finger in such a manner as to hold a pen, and
a placing wing portion (3b) which is formed on the outer periphery of the syringe holding portion (5), and is configured so as to allow a thumb to be placed on it in the case of holding the dental syringe (S) with three fingers that are the thumb, the forefinger and a middle finger in such a manner as to hold a pen,
wherein in a projected image on a plane orthogonal to an axial direction (AD) of the syringe (S),
an angle (θ) that is formed by a first line segment (L1) connecting a center (C) of a minimum virtual circle (V) that surrounds the outer periphery of the syringe holding portion (4) and a formation position (P1) of the clamping wing portion (3a), and a second line segment (L2) connecting the center (C) of the virtual circle (V) and a formation position (P2) of the placing wing portion (3b) is in a range from 90° to 170° inclusive.

2. The system according to claim 1,
wherein a maximum thickness dimension of the clamping wing portion (3a) is 0.2 mm to 5 mm inclusive.

3. The system according to claim 1 or 2,
wherein a maximum thickness dimension of the clamping wing portion (3a) and a maximum thickness dimension of the placing wing portion (3b) are 0.2 mm to 5 mm inclusive.

4. The system according to any one of claims 1 to 3,
wherein the dental syringe holder (1) holds the syringe (S) slidably.

5. The system according to any one of claims 1 to 4,
wherein the dental syringe holder (1) comprises only two wing portions consisting of the clamping wing portion (3a) and the placing wing portion (3b) as the wing portion.

6. The system according to any one of claims 1 to 5,
wherein the clamping wing portion (3a) and the placing wing portion (3b) are formed along the longitudinal direction of the syringe holding portion (4).

## Patentansprüche

1. System umfassend:
eine zahnärztliche Spritze (S); und
einen Halter für eine zahnärztliche Spritze (1), umfassend:
eine Spritzenhaltekomponente (4), die anbringbar und abnehmbar einen äußeren Zylinder der zahnärztlichen Spritze (S) hält; und
eine Klemmflügelkomponente (3a), die an der äußeren Peripherie der Spritzenhaltekomponente (5) ausgeformt ist, und so konzipiert ist, dass sie von einem Zeigefinger und einem Mittelfinger geklemmt wird, wenn die zahnärztliche Spritze (S) mit drei Fingern, die der Daumen, der Zeigefinger und der Mittelfinger sind, gehalten wird, wie auch ein Stift gehalten wird, und
eine Aufsetzflügelkomponente (3b), die an der äußeren Peripherie der Spritzenhaltekomponente (5) ausgeformt ist und so konzipiert ist, dass der Daumen auf sie aufgesetzt werden kann, wenn die zahnärztliche Spritze (S) mit drei Fingern, welche der Daumen, der Zeigefinger und der Mittelfinger sind, gehalten wird, wie auch ein Stift gehalten wird,
wobei in einem projizierten Bild auf eine Ebene orthogonal zu einer axialen Richtung (AD) der Spritze (S),
ein Winkel (θ), der gebildet wird durch ein erstes Liniensegment (L1), das einen Mittelpunkt (C) eines kleinstmöglichen virtuellen Kreises (V), der die äußere Peripherie der Spritzenhaltekomponente (4) umgibt, und eine Formationsposition (P1) der Klemmflügelkomponente (3a) verbindet, und ein zweites Liniensegment (L2), das den Mittelpunkt (C) des virtuellen Kreises (V) und eine Formationsposition (P2) der Aufsetzflügelkomponente (3b) verbindet, in einem Bereich von 90° bis 170° einschließlich liegt.

2. System nach Anspruch 1,
wobei eine maximale Dickenabmessung der Klemmflügelkomponente (3a) 0,2 mm bis 5 mm einschließlich beträgt.

3. System nach Anspruch 1 oder 2,
wobei eine maximale Dickenabmessung der Klemmflügelkomponente (3a) und eine maximale Dickenabmessung der Aufsetzflügelkomponente (3b) 0,2 mm bis 5 mm einschließlich betragen.

4. System nach einem der Ansprüche 1 bis 3,
wobei der Halter für eine zahnärztliche Spritze (1) die Spritze (S) verschiebbar hält.

5. System nach einem der Ansprüche 1 bis 4,
wobei der Halter für eine zahnärztliche Spritze (1) nur zwei Flügelkomponenten bestehend aus Klemmflügelkomponente (3a) und Aufsetzflügelkomponente (3b) als Flügelkomponenten umfasst.

6. System nach einem der Ansprüche 1 bis 5,
wobei die Klemmflügelkomponente (3a) und die Aufsetzflügelkomponente (3b) entlang der Längsrichtung der Spritzenhaltekomponente (4) ausgeformt sind.

## Revendications

1. Système comprenant:
une seringue dentaire (S); et
un support de seringue dentaire (1), comprenant:
une partie de maintien de seringue (4) qui maintient de manière attachable et amovible un cylindre extérieur de la seringue dentaire (S); et
une partie d'aile de serrage (3a) qui est formée sur une périphérie extérieure de la partie de maintien de seringue (5), et est configurée pour être serrée par un index et un majeur dans le cas où la seringue dentaire (S) est maintenue à trois doigts qui sont le pouce, l'index et le majeur, de la manière à laquelle on tient également un stylo, et
une partie d'aile de placement (3b) qui est formée sur la périphérie extérieure de la partie de maintien de seringue (5), et est configurée de manière à permettre à un pouce d'être placé sur celle-ci dans le cas où la seringue dentaire (S) est maintenue à trois doigts qui sont le pouce, l'index et le majeur, de la manière à laquelle on tient également un stylo,
dans lequel dans une image projetée sur un plan orthogonal à une direction axiale (AD) de la seringue (S),
un angle (θ) qui est formé par un premier segment de ligne (L1) reliant un centre (C) d'un cercle virtuel minimum (V) qui entoure la périphérie extérieure de la partie de maintien de seringue (4) et une position de formation (P1) de la partie d'aile de serrage (3a), et un deuxième segment de ligne (L2) reliant le centre (C) du cercle virtuel (V) et une position de formation (P2) de la partie d'aile de placement (3b) se situe dans une plage allant de 90° à 170° inclus.

2. Système selon la revendication 1,
dans lequel une dimension d'épaisseur maximale de la partie d'aile de serrage (3a) est comprise entre 0,2 mm et 5 mm inclus.

3. Système selon la revendication 1 ou 2,
dans lequel une dimension d'épaisseur maximale de la partie d'aile de serrage (3a) et une dimension d'épaisseur maximale de la partie d'aile de placement (3b) sont comprises entre 0,2 mm et 5 mm inclus.

4. Système selon l'une quelconque des revendications 1 à 3,
dans lequel le support de seringue dentaire (1) maintient la seringue (S) de manière coulissante.

5. Système selon l'une quelconque des revendications 1 à 4,
dans lequel le support de seringue dentaire (1) comprend seulement deux parties d'aile constituées de la partie d'aile de serrage (3a) et de la partie d'aile de placement (3b) en tant que parties d'aile.

6. Système selon l'une quelconque des revendications 1 à 5,
dans lequel la partie d'aile de serrage (3a) et la partie d'aile de placement (3b) sont formées le long de la direction longitudinale de la partie de maintien de seringue (4).
